# EUROPÄISCHE PATENTSCHRIFT

(11) **EP 1 654 246 B1**
(45) Veröffentlichungstag und Bekanntmachung des Hinweises auf die Patenterteilung: **15.11.2006**
(21) Anmeldenummer: 04766129.3
(22) Anmeldetag: 05.07.2004
(51) Int. Cl.: C07D 319/06, C07D 317/10

(54) **ACETALE, IHRE VERWENDUNG ALS RIECHSTOFFE UND VERFAHREN ZU IHRER HERSTELLUNG**
ACETALS, USE THEREOF AS FRAGRANCES AND METHODS FOR PRODUCTION THEREOF
ACETALS, LEUR UTILISATION COMME MATIERES ODORANTES ET LEUR PROCEDE DE PRODUCTION

(30) Priorität: 23.07.2003 DE 10333379
(43) Veröffentlichungstag der Anmeldung: 10.05.2006
(73) Patentinhaber: Symrise GmbH & Co. KG, 37603 Holzminden (DE)
(72) Erfinder: DILK, Erich, 37603 Holzminden (DE)
(74) Vertreter: Eisenführ, Speiser & Partner
(86) Internationale Anmeldenummer: PCT/EP2004/051360
(87) Internationale Veröffentlichungsnummer: WO 2005/009984

(56) Entgegenhaltungen:
- WO-A-00/04009
- US-A- 4 435 315
- US-A- 5 753 609
- BAUER, KURT; GARBE, DOROTHEA; SURBURG, HORST: "Common Fragrance and Flavor Materials" 2001, WILEY-VCH VERLAG GMBH , XP002309270 in der Anmeldung erwähnt Seiten 63-64; Seite 83

## Beschreibung

Die vorliegende Erfindung betrifft bestimmte Acetale und Acetalmischungen, deren Verwendung als Riechstoff, entsprechende Produkte sowie Verfahren zu deren Herstellung.

Wegen der im allgemeinen unzureichenden Verfügbarkeit vieler natürlicher Riechstoffkomponenten, der notwendigen Anpassung an wechselnde modische Geschmacksrichtungen sowie dem ständig steigenden Bedarf an neuen Riechstoffen, die allein oder in Form von Kompositionen wertvolle Duftstoffe bzw. Parfüms mit interessanten Duftnoten darstellen, besteht auch weiterhin ein Bedürfnis nach neuen Verbindungen mit wertvollen Riechstoffqualitäten. Gesucht sind insbesondere neue Riechstoffe, die über ihre geruchlichen Eigenschaften hinaus zusätzliche positive Sekundäreigenschaften, wie z.B. höhere Stabilität, höhere Ausgiebigkeit, besseres Haftungsvermögen usw. aufweisen.

Die vorliegende Erfindung betrifft gemäß einem ersten Aspekt Acetale der Formel I 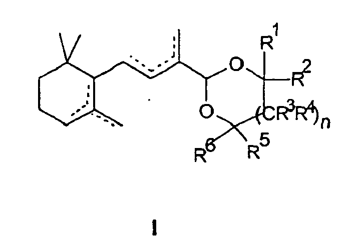 wobei
die Reste R¹ bis R⁶ unabhängig voneinander jeweils Wasserstoff, Methyl, Ethyl, n-Propyl, iso-Propyl, n-Butyl, iso-Butyl, sec-Butyl oder tert.-Butyl sind,
n = 0 oder 1 ist und
am Ort einer gestrichelt dargestellten Linie zwischen zwei C-Atomen eine Einfachbindung oder Doppelbindung vorliegt,
wobei eine gegebenenfalls vorhandene Doppelbindung in der den aliphatischen Ring mit der Acetal-Gruppe verbindenden Kette E- oder Z-konfiguriert ist,
mit der Maßgabe, dass
- die Zahl der Doppelbindungen, an denen ein C-Atom des aliphatischen Rings beteiligt ist, 0 oder 1 ist
- die Zahl der Doppelbindungen, an denen kein C-Atom des aliphatischen Rings beteiligt ist, 0 oder 1 ist.

Es können also insgesamt zwei Doppelbindungen vorhanden sein, von denen die eine dem aliphatischen Ring zugeordnet ist (Doppelbindung im Ring bzw. =CH₂ am Ring) und die andere nicht dem Ring zugeordnet ist (kein C-Atom des aliphatischen Rings ist an dieser Doppelbindung beteiligt; Doppelbindung liegt in der den aliphatischen Ring mit der Acetal-Gruppe verbindenden Kette oder die Kette trägt einen Substituenten =CH₂). Falls eine Doppelbindung in der den aliphatischen Ring mit der Acetal-Gruppe verbindenden Kette vorhanden ist, ist diese Doppelbindung wahlweise E- oder Z-konfiguriert. Formel I gibt somit keinen Hinweis zur E- oder Z-Konfiguration einer solchen Doppelbindung; die gewählte zeichnerische Darstellung wurde lediglich aus Gründen der Übersichtlichkeit gewählt und umfasst inhaltlich jeweils sämtliche möglichen Isomeren, Diastereomeren und Enantiomeren, insbesondere alle möglichen E- oder Z-Isomeren bei Vorhandensein einer Doppelbindung in der den aliphatischen Ring mit der Acetal-Gruppe verbindenden Kette.

Die erfindungsgemäßen Acetale sind insbesondere zur Verwendung als Riechstoffe geeignet, die in Parfümierungen eingesetzt werden können. Die erfindungsgemäßen Verbindungen besitzen überraschenderweise fruchtig-süße Geruchseigenschaften, wobei insbesondere Pflaumen- und Feigen-Noten dominieren. Zudem weisen die erfindungsgemäßen Acetale insbesondere in alkalischen und in oxidierenden Medien eine überraschend hohe, ganz hervorragende Stabilität auf. Insbesondere wegen dieser Eigenschaften eignen sich die erfindungsgemäßen Acetale in hervorragendem Maße für die Verwendung als Riechstoffe, und zwar insbesondere, wenn sie in einer Riechstoffmischung oder einem Parfüm bzw. einem parfümierten Produkt eingesetzt werden, die bzw. das einen pH > 7 besitzt und/oder oxidierend wirkt.

Aus dem Stand der Technik sind keine Geruchsbeschreibungen zu Verbindungen bekannt, die mit den erfindungsgemäßen Acetalen strukturell eng verwandt sind. Zum Aldehyd 2-Methyl-4-(2,6,6-trimethyl-1-cyclohexen-1-yl)-2-butenal, der als Edukt bei der Herstellung der erfindungsgemäßen Acetale eingesetzt werden kann (siehe unten) und von der Symrise GmbH & Co. KG unter der Bezeichnung "Boronal" vertrieben wird, wird folgende Geruchsbeschreibung angegeben: Floral, mit Veilchen-Akzenten und Nuancen von Heu, Kleie, Leder und Holz.

Die vielleicht auch noch als strukturell ähnlich zu bezeichnende Verbindung Beta-lonon wird in K. Bauer, D. Garbe und H. Surburg, Common Fragrance and Flavor Materials, 4th. Ed., Wiley-VCH, Weinheim 2001 wie folgt beschrieben: An Cedernholz erinnernd, Veilchen in der Verdünnung. Allgemein wird Beta-lonon als Veilchenriechstoff gesehen: Veilchen, irishaft, mit Cedernholznuancen.

Die erfindungsgemäßen Acetale besitzen somit Geruchseigenschaften, die mit denen des Boronal und des Beta-lonon nicht verwandt sind.

Vorzugsweise sind in den erfindungsgemäßen Acetalen die Reste R¹ - R⁶ unabhängig voneinander jeweils Wasserstoff oder Methyl. Besonders bevorzugt sind erfindungsgemäße Acetale der nachfolgenden Formel IA oder IB 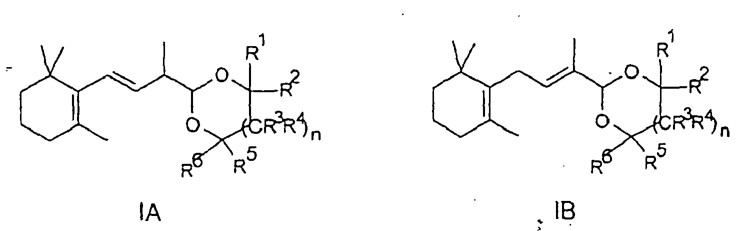 wobei die jeweilige Doppelbindung in der den aliphatischen Ring mit der Acetal-Gruppe verbindenden Kette E- oder Z-konfiguriert ist. Hinsichtlich der bevorzugten Bedeutung der Reste R¹ - R⁶ gilt dabei das zuvor Gesagte.

Ganz besonders bevorzugt sind die erfindungsgemäßen Acetale 2-[1-Methyl-3-(2,6,6-trimethyl-1-cyclohexen-1-yl)-allyl]- 1,3-Dioxolan und 2-[1-Methyl-3-(2,6,6-trimethyl-1-cyclohexen-1-yl)-propenyl]- 1,3-Dioxolan.

Die letztgenannten, besonders bevorzugten Acetale und andere erfindungsgemäße Acetale lassen sich durch Acetalisierung von 2-Methyl-4-(2,6,6-trimethyl-1-cyclohexen-1-yl)-2-butenal herstellen. Erfindungsgemäße Acetale, die auf diese Weise erhalten werden können, sind besonders bevorzugt. Hinsichtlich der bevorzugten Bedeutung der Reste R¹ - R⁶ und hinsichtlich der Konfiguration der Doppelbindung gilt dabei wieder das zuvor Gesagte.

Die besonders bevorzugten Acetale der Formeln IA und IB weisen Geruchsnoten auf, die einander ähneln, wobei jedoch die Verbindung der Formel IA insgesamt stärker und ausdrucksvoller als die Verbindung der Formel IB wirkt. Das Acetal der Formel IA ist deshalb besonders bevorzugt.

Die Erfindung betrifft auch Mischungen von zwei oder mehr erfindungsgemäßen Acetalen, wobei sämtliche Ausführungen zu besonders bevor zugten erfindungsgemäßen Acetalen auch hinsichtlich der Mischungen zutreffen. Insbesondere bevorzugt sind Acetalmischungen, in denen zumindest für zwei vorhandene Acetale die Reste R¹ - R⁶ unabhängig voneinander jeweils Wasserstoff oder Methyl sind. Ferner bevorzugt sind erfindungsgemäße Mischungen, in denen zumindest zwei der darin enthaltenen erfindungsgemäßen Acetale den Formeln IA bzw. IB entsprechen.

Ganz besonders bevorzugt ist eine Mischung, die 2-[1-Methyl-3-(2,6,6-trimethyl-1-cyclohexen-1-yl)-allyl]-1,3-Dioxolan und 2-[1-Methyl-3-(2,6,6-trimethyl-1-cyclohexen-1-yl)-propenyl]- 1,3-Dioxolan umfasst.

Sofern in einer erfindungsgemäßen Mischung Isomere der Formeln IA und IB nebeneinander vorliegen, ist es wegen der bevorzugten Geruchsnoten der Verbindungen der Formel IA bevorzugt, dass der Anteil an dem oder den Isomeren der Formel IA größer ist als der Anteil an dem oder den Isomeren der Formel IB.

Liegen in einer erfindungsgemäßen Mischung 2-[1-Methyl-3-(2,6,6-trimethyl-1-cydohexen-1-yl)-allyl]- 1,3-Dioxolan und 2-[1-Methyl-3-(2,6,6-trimethyl-1-cyciohexen-1-yl)-propenyl]-1,3-Dioxolan nebeneinander vor, so liegt dementsprechend vorteilhafterweise das 2-[1-Methyl-3-(2,6,6-trimethyl-1-cyclohexen-1-yl)-allyl]-1,3-Dioxolan in größerer Menge vor.

Die erfindungsgemäßen Acetale sowie deren Mischungen lassen sich nach an sich bekannten Syntheseverfahren der organischen Chemie erhalten. Vorteilhaft ist es, von 2-Methyl-4-(2,6,6-trimethyl-1-cyclohexen-1-yl)-2-butenal auszugehen und diese Verbindung durch Umsetzung mit einem aliphatischen 1,2- oder 1,3-Diol unter Säurekatalyse und Wasserabscheidung in das entsprechende erfindungsgemäße Acetal zu überführen. Die Substituenten am 1,2- oder 1,3-Diol bestimmen dabei, welche Bedeutung die Gruppen R¹ - R⁶ in der Formel I besitzen. Bei der genannten Reaktionsführung wird das gebildete Wasser vorteilhafterweise durch Destillation abgetrennt, insbesondere durch eine azeotrope Destillation und bevorzugt mittels einer azeotropen Destillation unter Verwendung eines Schleppmittels, um auf diese Weise eine hohe Produktausbeute zu erreichen. Als Schleppmittel sind insbesondere inerte Lösungsmittel wie Toluol, Xylol, Cyclohexan oder n-Pentan geeignet.

Erfindungsgemäße Mischungen von Acetalen der Formeln IA und IB lassen sich durch Wahl geeigneter Reaktionsbedingungen oder im Wege einer Umisomerisierung in unterschiedlichen Mengenverhältnissen herstellen.

Beispielsweise resultiert bei der Umsetzung von 2-Methyl-4-(2,6,6-trimethyl-1-cydohexen-1-yl)-2-butenal mit Ethylenglykol in Toluol ein Isomerenverhältnis für IA/IB von 6,5:1; wegen der Verwendung von Ethylenglykol entspricht hierbei die Verbindung IA 2-[1-Methyl-3-(2,6,6-trimethyl-1-cyclohexen-1-yl)-allyl]-1,3-Dioxolan, und die Verbindung IB entspricht 2-[1-Methyl-3-(2,6,6-trimethyl-1-cyclohexen-1-yl)-propenyl]-1,3-Dioxolan. Hingegen resultiert bei der Umsetzung von 2-Methyl-4-(2,6,6-trimethyl-1-cyclohexen-1-yl)-2-butenal mit Ethylenglykol in Cyclohexan ein Isomerenverhältnis für IA/IB von nur 2:1. Vgl. hierzu auch die Beispiele 1a) und 1c) weiter unten.

Auch die eingesetzte Menge an saurem Katalysator beeinflusst das Isomerenverhältnis; vgl. die Beispiele 1a) und 1b) weiter unten.

Durch Erhitzen eines Isomerengemisches mit einem hohen Anteil an einem Acetal der Formel IB und einem niedrigeren Anteil an einem Acetal der Formel IA mit p-Toluolsulfonsäure in Toluol kann beispielsweise im Wege der Umisomerisierung eine Verschiebung des Isomerenverhältnisses IA/IB von z. B. 1:2 zu 10:1 erreicht werden, d. h. zugunsten des Acetals der Formel IA; vgl. hierzu das Beispiel 1e) weiter unten. Abhängig von den jeweiligen Reaktionsbedingungen sind auch andere Umisomerisierungen möglich.

Die erfindungsgemäßen Acetale oder eine erfindungsgemäße Mischung von zwei oder mehr Acetalen kann, wie bereits erwähnt, in hervorragender Weise als Riechstoff oder zur Herstellung einer Riechstoffmischung oder eines Parfüms eingesetzt werden. Die erfindungsgemäßen Acetale oder Mischungen können auch vorteilhaft in Produkten enthalten sein, die einen Träger oder ein Substrat sowie eine damit in direkten Kontakt stehende sensorisch wirksame Menge des erfindungsgemäßen Acetals oder der erfindungsgemäßen Mischung von zwei oder mehr Acetalen umfassen.

Bevorzugte erfindungsgemäße Produkte sind ausgewählt aus der Gruppe bestehend aus: alkoholischen Parfüms, Körperpflegeprodukten und im Haushalt zu verwendenden Reinigungs- oder Pflegeprodukten. Dabei sind die Körperpflegeprodukte vorzugsweise ausgewählt aus der Gruppe bestehend aus Seifen, Duschgelen, Shampoos, Badezusätzen, Hautcremes, Körperlotionen und Deodorantien, und die Reinigungsmittel sind vorzugsweise ausgewählt aus der Gruppe bestehend aus WaschMitteln, Wäscheweichspülem, Raumluftverbesserern und Reinigern.

Die erfindungsgemäßen Acetale und die erfindungsgemäßen Mischungen lassen sich mit anderen Riechstoffen in verschiedenen, unterschiedlichen Mengenverhältnissen zu neuartigen Parfümkompositionen kombinieren.

Beispiele für Riechstoffe, mit denen die erfindungsgemäßen Acetale vorteilhaft kombiniert werden können, finden sich z.B. in S. Arctander, Perfume and Flavor Materials, Vol. I und II, Montclair, N. J., 1969, Selbstverlag oder K. Bauer, D. Garbe und H. Surburg, Common Fragrance and Flavor Materials, 4^{th}. Ed., Wiley-VCH, Weinheim 2001.

Im einzelnen seien genannt:

Extrakte aus natürlichen Rohstoffen wie Etherische Öle, Concretes, Absolues, Resine, Resinoide, Balsame, Tinkturen wie z. B.

Ambratinktur; Amyrisöl; Angelicasamenöl; Angelicawurzelöl; Anisöl; Baldrianöl; Basilikumöl; Baummoos -Absolue; Bayöl; Beifußöl; Benzoeresin; Bergamotteöl; Bienenwachs-Absolue; Birkenteeröl; Bittermandelöl; Bohnenkrautöl; Buccoblätteröl; Cabreuvaöl; Cadeöl; Calmusöl; Campheröl; Canangaöl; Cardamomenöl; Cascarillaöl; Cassiaöl; Cassie-Absolue; Castoreum-absolue; Cedernblätteröl; Cedernholzöl; Cistusöl; Citronellöl; Citronenöl; Copaivabalsam ; Copaivabalsamöl; Corianderöl; Costuswurzelöl; Cuminöl; Cypressenöl; Davanaöl; Dillkrautöl; Dillsamenöl; Eau de brouts-Absolue; Eichenmoos-Absolue; Elemiöl; Estragonöl; Eucalyptus-citriodora-Öl; Eucalyptusöl; Fenchelöl ; Fichtennadelöl; Galbanumöl; Galbanumresin; Geraniumöl; Grapefruitöl; Guajakholzöl; Gurjunbalsam; Gurjunbalsamöl; Helichrysum-Absolue; Helichrysumöl; Ingweröl; Iriswurzel-Absolue; lriswurzelöl; Jasmin-Absolue; Kalmusöl; Kamillenöl blau; Kamillenöl römisch; Karottensamenöl; Kaskarillaöl; Kiefernadelöl; Krauseminzöl; Kümmelöl; Labdanumöl; Labdanum-Absolue; Labdanumresin; Lavandin-Absolue; Lavandinöl ; Lavendel-Absolue; Lavendelöl; Lemongrasöl; Liebstocköl; Limetteöl destilliert; Limetteöl gepreßt; Linaloeöl; Litsea-cubeba-Öl; Lorbeerblätteröl; Macisöl; Majoranöl; Mandarinenöl; Massoirindenöl; Mirnosa-Absolue; Moschuskömeröl; Moschustinktur; Muskateller-Salbei-Öl; Muskatnußöl; Myrrhen-Absolue; Myrrhenöl; Myrtenöl; Nelkenblätteröl; Nelkenblütenöl; Neroliöl; Olibanum-Absolue; Olibanumöl; Opopanaxöl; Orangenblüten-Absolue; Orangenöl; Origanumöl; Palmarosaöl; Patchouliöl; Perillaöl; Perubalsamöl; Petersilienblätteröl; Petersiliensamenöl; Petitgrainöl; Pfefferminzöl; Pfefferöl; Pimentöl; Pineöl; Poleyöl; Rosen-Absolue; Rosenholzöl; Rosenöl; Rosmarinöl; Salbeiöl dalmatinisch; Salbeiöl spanisch; Sandelholzöl; Selleriesamenöl; Spiklavendelöl; Sternanisöl; Styraxöl; Tagetesöl; Tannennadelöl; Tea-tree-Öl; Terpentinöl; Thymianöl; Tolubalsam; Tonka-Absolue; Tuberosen-Absolue; Vanilleextrakt; Veilchenblätter-Absolue; Verbenaöl; Vetiveröl; Wacholderbeeröl; Weinhefenöl; Wermutöl; Wintergrünöl; Ylangöl; Ysopöl; Zibet-Absolue; Zimtblätteröl; Zimtrindenöl, sowie Fraktionen davon, bzw. daraus isolierten Inhaltsstoffen;

Einzel-Riechstoffe aus der Gruppe
der Kohlenwasserstoffe, wie z. B. 3-Caren; α-Pinen; β-Pinen; α-Terpinen; γ-Terpinen; p-Cymol; Bisabolen; Camphen; Caryophyllen; Cedren; Farnesen; Limonen; Longifolen; Myrcen; Ocimen; Valencen; (E,Z)-1,3,5-Undecatrien; Styrol; Diphenylmethan;
der aliphatischen Alkohole wie z. B. Hexanol; Octanol; 3-Octanol; 2,6-Dimethylheptanol; 2-Methyl-2-heptanol; 2-Methyl-2-octanol; (E)-2-Hexenol; (E)- und (Z)-3-Hexenol; 1-Octen-3-ol; Gemisch von 3,4,5,6,6-Pentamethyl-3/4-hepten-2-ol und 3,5,6,6-Tetramethyl-4-methyleneheptan-2-ol; (E,Z)-2,6-Nonadienol; 3,7-Dimethyl-7-methoxyoctan-2-ol; 9-Decenol; 10-Undecenol; 4-Methyl-3-decen-5-ol;
der aliphatischen Aldehyde und deren Acetale wie z. B. Hexanal; Heptanal; Octanal; Nonanal; Decanal; Undecanal; Dodecanal; Tridecanal; 2-Methyloctanal; 2-Methylnonanal; (E)-2-Hexenal; (Z)-4-Heptenal; 2,6-Dimethyl-5-heptenal; 10-Undecenal; (E)-4-Decenal; 2-Dodecenal; 2,6,10-Trimethyl-5,9-undecadienal; Heptanaldiethylacetal; 1,1-Dimethoxy-2,2,5-trimethyl-4-hexen; Citronellyloxyacetaldehyd;
der aliphatischen Ketone und deren Oxime wie z. B. 2-Heptanon; 2-Octanon; 3-Octanon; 2-Nonanon; 5-Methyl-3-heptanon ; 5-Methyl-3-heptanonoxim; 2,4,4,7-Tetramethyl-6-octen-3-on; 6-Methyl-5-hepten-2-on;
der aliphatischen schwefelhaltigen Verbindungen wie z. B.; 3-Methylthiohexanol; 3-Methylthiohexylacetat; 3-Mercaptohexanol; 3-Mercaptohexylacetat; 3-Mercaptohexylbutyrat; 3-Acetylthiohexylacetat; 1-Menthen-8-thiol;
der aliphatischen Nitrile wie z.B.; 2-Nonensäurenitril; 2-Tridecensäurenitril; 2,12-Tridecadiensäurenitril; 3,7-Dimethyl-2,6-octadiensäurenitril, 3,7-Dimethyl-6-octensäurenitril;
der aliphatischen Carbonsäuren und deren Ester wie z.B. (E)- und (Z)-3-Hexenylformiat; Ethylacetoacetat; Isoamylacetat; Hexylacetat; 3,5,5-Trimethylhexylacetat; 3-Methyl-2-butenylacetat; (E)-2-Hexenylacetat; (E)- und (Z)-3-Hexenylacetat; Octylacetat; 3-Octylacetat; 1-Octen-3-ylacetat; Ethylbutyrat; Butylbutyrat, ; Isoamylbutyrat; Hexylbutyrat; (E)-und (Z)-3-Hexenylisobutyrat; Hexylcrotonat; Ethylisovalerianat; Ethyl-2-methylpentanoat; Ethylhexanoat; Allylhexanoat; Ethylheptanoat; Allylheptanoat; Ethyloctanoat; Ethyl-(E,Z)-2,4-decadienoat; Methyl-2-octinat; Methyl-2-noninat; Allyl-2-isoamyloxyacetat; Methyl-3,7-dimethyl-2,6-octadienoat;
der acyclischen Terpenalkohole wie z. B. Citronellol; Geraniol; Nerol; Linalool; Lavadulol; Nerolidol; Farnesol; Tetrahydrolinalool; Tetrahydrogeraniol; 2,6-Dirnethyl-7-octen-2-ol; 2,6-Dimethyloctan-2-ol; 2-Methyl-6-methyten-7-octen-2-ol; 2,6-Dimethyl-5,7-octadien-2-ol; 2,6-Dimethyl-3,5-octadien-2-ol; 3,7-Dimethyl-4,6-octadien-3-ol; 3,7-Dimethyl-1,5,7-octatrien-3-ol 2,6-Dimethyl-2,5,7-octatrien-1-ol; sowie deren Formiate, Acetate, Propionate, Isobutyrate, Butyrate, Isovalerianate, Pentanoate, Hexanoate, Crotonate, Tiglinate, 3-Methyl-2-butenoate;
der acyclischen Terpenaldehyde und -ketone wie z. B.; Geranial; Neral; Citronellal; 7-Hydroxy-3,7-dimethyloctanal; 7-Methoxy-3,7-dimethyloctanal; 2,6,10-Trimethyl-9-undecenal; Geranylaceton; sowie die Dimethyl- und Diethylacetale von Geranial, Neral, 7-Hydroxy-3,7-dimethyloctanal;
der cyclischen Terpenalkohole wie z. B. Menthol; Isopulegol; alpha-Terpineol; Terpinenol-4; Menthan-8-ol; Menthan-1-ol; Menthan-7-ol; Borneol; Isoborneol; Linalooloxid; Nopol; Cedrol; Ambrinol; Vetiverol; Guajol; sowie deren Formiate, Acetate, Propionate, Isobutyrate, Butyrate, Isovalerianate, Pentanoate, Hexanoate, Crotonate, Tiglinate, 3-Methyl-2-butenoate;
der cyclischen Terpenaldehyde und -ketone wie z. B.:; Menthon; Isomenthon ; 8-Mercaptomenthan-3-on ; Carvon; Campher; Fenchon; alpha-lonon; beta-Ionon; alpha-n-Methylionon; beta-n-Methylionon; alpha-Isomethylionon; beta-Isomethylionon; alpha-lron; alpha-Damascon; beta-Damascon; beta-Damascenon; delta-Damascon; gamma-Damascon; 1-(2,4,4-Trimethyl-2-cyclohexen-1-yl)-2-buten-1-on ; 1,3,4,6,7,8a-Hexahydro-1,1,5,5-tetramethyl-2H-2,4a-methanonaphthalen-8(5H)-on; Nootkaton ; Dihydronootkaton ; alpha-Sinensal ; beta-Sinensal ; Acetyliertes Cedemholzöl (Methylcedrylketon);
der cyclischen Alkohole wie z.B. 4-tert.-Butylcydohexanol ; 3,3,5-Trimethylcyclohexanol; 3-Isocamphylcyclohexanol; 2,6,9-Trimethyl-Z2,Z5,E9-cyclododecatrien-1-ol; 2-Isobutyl-4-methyltetrahydro-2H-pyran-4-ol;
der cycloaliphatischen Alkohole wie z.B. alpha,3,3-Trimethylcyclohexylmethanol; 2-Methyl-4-(2,2,3-trimethyl-3-cydopent-1-yl)butanol; 2-Methyl-4-(2,2,3-trimethyl-3-cyclopent-1-yl)-2-buten-1-ol; 2-Ethyl-4-(2,2,3-trimetfiyl-3-cydopent-1-yl)-2-buten-1-ol; 3-Methyl-5-(2,2,3-trimethyl-3-cydopent-1-yl)-pentan-2-ol; 3-Methyl-5-(2,2,3-trimethyl-3-cydopent-1-yl)-4-penten-2-ol; 3,3-Dimethyl-5-(2,2,3-trimethyl-3-cyclopent-1-yl)-4-penten-2-ol; 1-(2,2,6-Trimethylcyclohexyl)pentan-3-ol; 1-(2,2,6-Trimethylcydohexyl)hexan-3-ol,
der cyclischen und cydoaliphatischen Ether wie z.B. Cineol; Cedrylmethylether; Cyclododecylmethylether; (Ethoxymethoxy)cydododecan; alpha-Cedrenepoxid; 3a,6,6,9a-Tetramethyldodecahydronaphtho[2,1-b]furan; 3a-Ethyl-6,6,9a-trimethyldodecahydronaphtho[2,1-b]furan; 1,5,9-Trimethyl-13-oxabicyclo[10.1.0]trideca-4,8-dien; Rosenoxid; 2-(2,4-Dimethyl-3-cydohexen-1-yl)-5-methyl-5-(1-methylpropyl)-1,3-dioxan;
der cyclischen und makrocyclischen Ketone wie z.B. ; 4-tert.-Butylcydohexanon; 2,2,5-Trimethyl-5-pentylcyclopentanon; 2-Heptylcydopentanon; 2-Pentylcyclopentanon; 2-Hydroxy-3-methyl-2-cydopenten-1-on; 3-Methyl-cis-2-penten-1-yl-2-cyclopenten-1-on; 3-Methyl-2-pentyl-2-cyclopenten-1-on; 3-Methyl-4-cyclopentadecenon; 3-Methyl-5-cyclopentadecenon; 3-Methylcyclopentadecanon; 4-(1-Ethoxyvinyl)-3,3,5,5-tetramethylcyclohexanon; 4-tert.-Pentylcyclohexanon; 5-Cyclohexadecen-1-on; 6,7-Dihydro-1,1,2,3,3-pentamethyl-4(5H)-indanon; 8-Cyclohexadecen-1-on; 9-Cydoheptadecen-1-on; Cyclopentadecanon; Cyclohexadecanon;
der cycloaliphatischen Aldehyde wie z.B. ; 2,4-Dimethyl-3-cyclohexencarbaldehyd; 2-Methyl-4-(2,2,6-trimethyl-cydohexen-1-yl)-2-butenal; 4-(4-Hydroxy-4-methylpentyl)-3-cyclohexencarbaldehyd; 4-(4-Methyl-3-penten-1-yl)-3-cyclohexencarbaldehyd;
der cycloaliphatischen Ketone wie z. B. ; 1-(3,3-Dimethylcyclohexyl)-4-penten-1-on; 1-(5,5-Dimethyl-1-cyclohexen-1-yl)-4-penten-1-on; 2,3,8,8-Tetramethyl-1,2,3,4,5,6,7,8-octahydro-2-naphthalenylmethylketon; Methyl-2,6,10-trimethyl-2,5,9-cyclododecatrienylketon; tert.-Butyl-(2,4-dirnethyl-3-cyclohexen-1-yl)keton;
der Ester cyclischer Alkohole wie z.B. ; 2-tert-Butylcyclohexylacetat; 4-tert-Butylcyclohexylacetat; 2-tert-Pentylcyclohexylacetat; 4-tert-Pentylcyclohexylacetat; Decahydro-2-naphthylacetat; 3-Pentyltetrahydro-2H-pyran-4-ylacetat; Decahydro-2,5,5,8a-tetramethyl-2-naphthylacetat; 4,7-Methano-3a,4,5,6,7,7a-hexahydro-5, bzw. 6-indenylacetat; 4,7-Methano-3a,4,5,6,7,7a-hexahydro-5, bzw. 6-indenylpropionat; 4,7-Methano-3a,4,5,6,7,7a-hexahydro-5, bzw. 6-indenylisobutyrat; 4,7-Methanooctahydro-5, bzw. 6-indenylacetat;
der Ester cycloaliphatischer Carbonsäuren wie z. B. ; Allyl-3-cyclohexylpropionat; Allylcyclohexyloxyacetat; cis- und trans-Methyldihydrojasmonat; cis- und trans-Methyljasmonat; Methyl-2-hexyl-3-oxocyclopentancarboxylat; Ethyl-2-ethyl-6,6-dimethyl-2-cyclohexencarboxylat; Ethyl-2,3,6,6-tetramethyl-2-cyclohexencarboxylat; Ethyl-2-methyl-1,3-dioxolan-2-acetat;
der araliphatischen Alkohole wie z.B. ; Benzylalkohol; 1-Phenylethylalkohol; 2-Phenylethylalkohol; 3-Phenylpropanol; 2-Phenylpropanol; 2-Phenoxyethanol; 2,2-Dimethyl-3-phenylpropanol; 2,2-Dimethyl-3-(3-methylphenyl)propanol; 1,1-Dimethyl-2-phenylethylalkohol; 1,1-Dimethyl-3-phenylpropanol; 1-Ethyl-1-methyl-3-phenylpropanol; 2-Methyl-5-phenylpentanol; 3-Methyl-5-phenylpentanol; 3-Phenyl-2-propen-1-ol; 4-Methoxybenzylalkohol; 1-(4-Isopropylphenyl)ethanol;
der Ester von araliphatischen Alkoholen und aliphatischen Carbonsäuren wie z.B. ; Benzylacetat; Benzylpropionat; Benzylisobutyrat; Benzylisovalerianat; 2-Phenylethylacetat; 2-Phenylethylpropionat; 2-Phenylethylisobutyrat; 2-Phenylethylisovalerianat; 1-Phenylethylacetat; alpha-Trichlormethylbenzylacetat; alpha,alpha-Dimethylphenylethylacetat; alpha,alpha-Dimethylphenylethylbutyrat; Cinnamylacetat; 2-Phenoxyethylisobutyrat; 4-Methoxybenzylacetat;
der araliphatischen Ether wie z.B. 2-Phenylethylmethylether; 2-Phenylethylisoamylether; 2-Phenylethyl-1-ethoxyethylether; Phenylacetaldehyddimethylacetal; Phenylacetaldehyddiethylacetal; Hydratropaaldehyddimethylacetal; Phenylacetaldehydglycerinacetal; 2,4,6-Trimethyl-4-phenyl-1,3-dioxane; 4,4a,5,9b-Tetrahydroindeno[1,2-d]-m-dioxin; 4,4a,5,9b-Tetrahydro-2,4-dimethylindeno[1,2-d]-m-dioxin;
der aromatischen und araliphatischen Aldehyde wie z. B. Benzaldehyd; Phenylacetaldehyd; 3-Phenylpropanal; Hydratropaaldehyd; 4-Methylbenzaldehyd; 4-Methylphenylacetaldehyd; 3-(4-Ethylphenyl)-2,2-dimethylpropanal; 2-Methyl-3-(4-isopropylphenyl)propanal; 2-Methyl-3-(4-tert.-butylphenyl)propanal; 3-(4-tert.-Butylphenyl)propanal; Zimtaldehyd; alpha-Butylzimtaldehyd; alpha-Amylzimtaldehyd; alpha-Hexylzimtaldehyd; 3-Methyl-5-phenylpentanal; 4-Methoxybenzaldehyd; 4-Hydroxy-3-methoxybenzaldehyd; 4-Hydroxy-3-ethoxybenzaldehyd; 3,4-Methylendioxybenzaldehyd; 3,4-Dimethoxybenzaldehyd; 2-Methyl-3-(4-methoxyphenyl)propanal; 2-Methyl-3-(4-methylendioxyphenyl)propanal;
der aromatischen und araliphatischen Ketone wie z.B. Acetophenon; 4-Methylacetophenon; 4-Methoxyacetophenon; 4-tert.-Butyl-2,6-dimethylacetophenon; 4-Phenyl-2-butanon; 4-(4-Hydroxyphenyl)-2-butanon; 1-(2-Naphthalenyl)ethanon; Benzophenon; 1,1,2,3,3,6-Hexamethyl-5-indanylmethylketon; 6-tert.-Butyl-1,1 -dimethyl-4-indanylmethylketon; 1-[2,3-dihydro-1,1,2,6-tetramethyl-3-(1-methylethyl)-1H-5-indenyl]ethanon; 5',6',7',8'-Tetrahydro-3',5',5',6',8',8'-hexamethyl-2-acetonaphthon;
der aromatischen und araliphatischen Carbonsäuren und deren Ester wie z.B. Benzoesäure; Phenylessigsäure; Methylbenzoat; Ethylbenzoat; Hexylbenzoat; Benzyl-benzoat; Methylphenylacetat; Ethylphenylacetat; Geranylphenylacetat; Phenylethyl-phenylacetat; Methylcinnamat; Ethylcinnamat; Benzylcinnamat; Phenylethylcinnamat; Cinnamylcinnamat; Allylphenoxyacetat; Methylsalicylat; Isoamylsalicylat; Hexylsalicylat; Cyclohexylsalicylat; Cis-3-Hexenylsalicylat; Benzylsalicylat; Phenylethylsalicylat; Methyl-2,4-dihydroxy-3,6-dimethylbenzoat; Ethyl-3-phenylglycidat; Ethyl-3-methyl-3-phenylglycidat;
der stickstoffhaltigen aromatischen Verbindungen wie z.B. 2,4,6-Trinitro-1,3-dimethyl-5-tert.-butylbenzol; 3,5-Dinitro-2,6-dimethyl-4-tert.-butylacetophenon; Zimtsäurenitril; 5-Phenyl-3-methyl-2-pentensäurenitril; 5-Phenyl-3-methylpentansäurenitril; Methylanthranilat; Methy-N-methylanthranilat; Schiffsche Basen von Methylanthranilat mit 7-Hydroxy-3,7-dimethyloctanal, 2-Methyl-3-(4-tert-butylphenyl)propanal oder 2,4-Dimethyl-3-cyclohexencarbaldehyd; 6-lsopropylchinolin; 6-lsobutylchinolin; 6-sec.-Butylchinolin; Indol; Skatol; 2-Methoxy-3-isopropylpyrazin; 2-Isobutyl-3-methoxypyrazin;
der Phenole, Phenylether und Phenylester wie z.B. Estragol; Anethol; Eugenol; Eugenylmethylether; Isoeugenol; Isoeugenylmethylether; Thymol; Carvacrol; Diphenylether; beta-Naphthylmethylether; beta-Naphthylethylether; beta-Naphthylisobutylether; 1,4-Dimethoxybenzol; Eugenylacetat; 2-Methoxy-4-methylphenol; 2-Ethoxy-5-(1-propenyl)phenol; p-Kresylphenylacetat;
der heterocyclischen Verbindungen wie z.B. 2,5Dimethyl-4-hydroxy-2H-furan-3-on; 2-Ethyl-4-hydroxy-5-methyl-2H-furan-3-on; 3-Hydroxy-2-methyl-4H-pyran-4-on; 2-Ethyl-3-hydroxy-4H-pyran-4-on;
der Lactone wie z.B. 1,4-Octanolid; 3-Methyl-1,4-octanolid; 1,4-Nonanolid; 1,4-Decanolid; 8-Decen-1,4-olid; 1,4-Undecanolid; 1,4-Dodecanolid; 1,5-Decanolid; 1,5-Dodecanolid; 1,15-Pentadecanolid; cis- und trans-11-Pentadecen-1,15-olid; cis- und trans-12-Pentadecen-1,15-olid; 1,16-Hexadecanolid; 9-Hexadecen-1,16-olid; 10-Oxa-1,16-hexadecanolid; 11-Oxa-1,16-hexadecanolid; 12-Oxa-1,16-hexadecanolid; Ethylen-1,12-dodecandioat; Ethylen-1,13-tridecandioat; Cumarin; 2,3-Dihydrocumarin; Octahydrocumarin.;

In Parfümkompositionen beträgt die eingesetzte Gesamtmenge an erfindungsgemäßen Acetalen vorteilhafterweise 0,05 bis 50 Gew.-%, vorzugsweise 0,5 bis 20 Gew.-%, bezogen auf die gesamte Parfümöl-Komposition.

Erfindungsgemäße Acetale enthaltende Parfümöle können in flüssiger Form, unverdünnt oder mit einem Lösungmittel verdünnt für Parfümierungen eingesetzt werden. Geeignete Lösungsmittel hierfür sind z.B. Ethanol, Isopropanol, Diethylenglycolmonoethylether, Glycerin, Propylenglycol, 1,2-Butylenglycol, Dipropylenglycol, Diethylphthalat, Triethylcitrat, Isopropylmyristat usw.

Des weiteren können erfindungsgemäße Acetale enthaltende Parfümöle an einem Trägerstoff adsorbiert sein, der sowohl für eine feine Verteilung der Riechstoffe im Produkt als auch für eine kontrollierte Freisetzung bei der Anwendung sorgt. Derartige Träger können poröse anorganische Materialien wie Leichtsulfat, Kieselgele, Zeolithe, Gipse, Tone, Tongranulate, Gasbeton usw. oder organische Materialien wie Hölzer und Cellulose-basierende Stoffe sein.

Erfindungsgemäße Acetale enthaltende Parfümöle können auch mikroverkapselt, sprühgetrocknet, als Einschluß-Komplexe oder als Extrusions-Produkte vorliegen und in dieser Form dem zu parfümierenden Produkt hinzugefügt werden.

Gegebenenfalls können die Eigenschaften der derart modifizierten Parfümöle durch sog "Coaten" mit geeigneten Materialien im Hinblick auf eine gezieltere Duftfreisetzung weiter optimiert werden, wozu vorzugsweise wachsartige Kunststoffe wie z.B. Polyvinylalkohol verwendet werden.

Die Mikroverkapselung der Parfümöle kann beispielsweise durch das sogenannte Koazervationsverfahren mit Hilfe von Kapselmaterialien z.B. aus polyurethan-artigen Stoffen oder Weichgelatine, erfolgen. Die sprühgetrockneten Parfümöle können beispielsweise durch Sprühtrocknung einer das Parfümöl enthaltenden Emulsion, bzw. Dispersion hergestellt werden, wobei als Trägerstoffe modifizierte Stärken, Proteine, Dextrin und pflanzliche Gummen verwendet werden können. Einschluß-Komplexe können z.B. durch Eintragen von Dispersionen von dem Parfümöl und Cyclodextrinen oder Harnstoffderivaten in ein geeignetes Lösungsmittel, z.B. Wasser, hergestellt werden. Extrusions-Produkte können durch Verschmelzen der Parfümöle mit einem geeigneten wachsartigen Stoff und durch Extrusion mit nachfolgender Erstarrung, ggf. in einem geeigneten Lösungsmittel, z.B. lsopropanol, hergestellt werden.

Erfindungsgemäße Acetale enthaltende Parfümöle können in konzentrierter Form, in Lösungen oder in oben beschriebener modifizierter Form verwendet werden für die Herstellung von z.B. Parfüm-Extraits, Eau de Parfums, Eau de Toilettes, Rasierwässer, Eau de Colognes, Pre-shave-Produkte, Splash-Colognes und parfümierten Erfrischungstüchern sowie die Parfümierung von sauren, alkalischen und neutralen Reinigungsmitteln, wie z.B. Fußbodenreinigem, Fensterglasreinigem, Geschirrspülmittel, Bad- und Sanitärreinigem, Scheuermilch, festen und flüssigen WC-Reinigem, pulver- und schaumförmigen Teppichreinigern, flüssigen Waschmitteln, pulverförmigen Waschmitteln, Wäschevorbehandlungsmitteln wie Bleichmittel, Einweichmittel und Fleckenentfemern, Wäscheweichspülem, Waschseifen, Waschtabletten, Desinfektionsmitteln, Oberflächendesinfektionsmittein sowie von Luftverbesserern in flüssiger, gelartiger oder auf einem festen Träger aufgebrachter Form, Aerosolsprays, Wachsen und Polituren wie Möbelpolituren, Fußbodenwachsen, Schuhcremes sowie Körperpflegemitteln wie z.B. festen und flüssigen Seifen, Duschgelen, Shampoos, Rasierseifen, Rasierschäumen, Badeölen, kosmetischen Emulsionen vom Öl-in-Wasser-, vom Wasser-in-Öl- und vom Wasser-in-Öl-in-Wasser-Typ wie z.B. Hautcremes- und -lotionen, Gesichtscremes und -lotionen, Sonnenschutzcremes-und -lotionen, After-sun-cremes und -lotionen, Handcremes und -lotionen, Fußcremes und -lotionen, Enthaarungscremes und -lotionen, After-shave-Cremes und -lotionen, Bräunungscremes und -lotionen, Haarpflegeprodukten wie z.B. Haarsprays, Haargelen, festigenden Haarlotionen, Haarspülungen, permanenten und semipermanenten Haarfärbemitteln, Haarverformungsmitteln wie kaltwellen und Haarglättungsmitteln, Haarwässern, Haarcremes und -lotionen, Deodorantien und Antiperspirantien wie z.B. Achselsprays, Roll-ons, Deosticks, Deocremes, Produkten der dekorativen Kosmetik wie z.B. Lidschatten, Nagellacke, Make-ups, Lippenstifte, Mascara sowie von Kerzen, Lampenölen, Räucherstäbchen, Insektiziden, Repellentien, Treibstoffen.

Die nachfolgenden Beispiele sollen die Erfindung (in ihren verschiedenen Aspekten) näher erläutern:

### Beispiele:

### Beispiel 1

### Herstellung eines lsomerengemischs aus 2-[1-Methyl-3-(2,6,6-trimethyl-1-cyclohexen-1-yl)-allyl]-1,3-Dioxolan (Isomer 1) und 2-[1-Methyl-3-(2,6,6-trimethyl-1-cyclohexen-1-yl)-propenyl]-1,3-Dioxolan (Isomer 2)

a) Reaktionsparameter: Schleppmittel: Toluol; Menge p-Toluolsulfonsäure = 1 g
Molverhältnis 2-Methyl-4-(2.6.6-trimethyl-1-cyclohexen-1-yl)-2-butenal:Ethylenglykol = 1:3
103g (0,5 Mol) 2-Methyl-4-(2.6.6-trimethyl-1-cyclohexen-1-yl)-2-butenal, 93g (1,5 Mol) Ethylenglykol und 1 g p-Toluolsulfonsäure werden mit 200 g Toluol 2,5 Stunden am Wasserabscheider erhitzt. Nach dem Abkühlen wird mit Sodalösung gewaschen und über Natriumsulfat getrocknet. Man fügt 3 g Soda hinzu und destilliert an einer 20-cm Füllkörperkolonne. Bei 122-132°C/2,3-2,1 mbar resultieren 99 g Produkt, das zu 54% aus Isomer 1 und zu 8,3% Isomer 2 besteht, und das weitere Doppelbindungsisomere enthält.
Geruch: Pflaume, Feige, Aprikose, Damascon, angenehm fruchtig-süße Kombination
Zur Strukturbestimmung wurden die Isomere 1 und 2 durch Hochdruckflüssig-Chromatographie getrennt und mittels NMR-Spektroskopie analysiert.
2-[1-Methyl-3-(2,6,6-trimethyl-1-cyclohexen-1-yl)-allyl]-1,3-Dioxolan (Isomer 1)

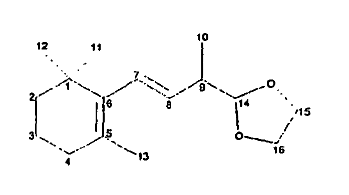

| δ(ppm) | | | J (Hz) | |
|---|---|---|---|---|
| 0,980 | s | 3H | | CH3 (11 oder 12) |
| 0,982 | s | 3H | | CH3 (11 oder 12) |
| 1,10 | d | 3H | 7,0 | CH3 (10) |
| 1,43 | m | 2H | | CH2 (2) |
| 1,59 | m | 2H | | CH2 (3) |
| 1,67 | q | 3H | 0,9 | CH3 (13) |
| 1,96 | t br | 2H | 6,4 | CH2 (4) |
| 2,50 | ddqd | 1H | 1,1/4,1/6,9/8,0 | CH (9) |
| 3,86 | m | 2H | | CH2 (15, 16) |
| 3,95 | m | 2H | | CH2 (15,16) |
| 4,77 | d | 1H | 4,2 | CH (14) |
| 5,36 | dd | 1H | 7,8/ 16,0 | CH (8) |
| 5,91 | d br | 1H | 16,0 | CH (7) |

Lösungsmittel: CDCl3
interner Standard : Tetramethylsilan (TMS)
Frequenz: 400 MHz
2-[1-Methyl-3-(2,6,6-trimethyl-1-cydohexen-1-yl)-propenyl]-1,3-Dioxolan (Isomer 2)

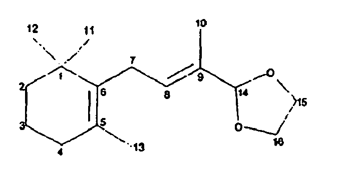

| δ(ppm) | | | J (Hz) | |
|---|---|---|---|---|
| 0,96 | s | 6H | | 2 *CH3 (11,12) |
| 1,42 | m | 2H | | CH2 (2) |
| 1,53 | s br | 3H | | CH3 (13) |
| 1,57 | m | 2H | | CH2 (3) |
| 1,67 | dt | 3H | 1,4/1,1 | CH3 (10) |
| 1,91 | t | 2H | 6,3 | CH2 (4) |
| 2,78 | d | 2H | 6,4 | CH2 (7) |
| 3,90 | m | 2H | | CH2 (15,16) |
| 4,01 | m | 2H | | CH2 (15,16) |
| 5,08 | d | 1H | 0,6 | CH (14) |
| 5,48 | dqt | 1H | 0,6/1,4/6,4 | CH (8) |

Lösungsmittel: CDCl3
interner Standard : Tetramethylsilan (TMS)
Frequenz: 400 MHz
b) Reaktionsparameter: Schleppmittel: Toluol; Menge p-Toluolsulfonsäure = 0,15 g
Molverhältnis 2-Methyl-4-(2.6.6-trimethyl-1-cydohexen-1-yl)-2-butenal:Ethylenglykol = 1:3
103g (0,5 Mol) 2-Methyl-4-(2.6.6-trimethyl-1-cyclohexen-1-yl)-2-butenal, 93 g (1,5 Mol) Ethylenglykol und 0,15 g p-Toluolsulfonsäure werden mit 200 g Toluol 2,5 Stunden am Wasserabscheider erhitzt. Hierbei werden 39 g eines Wasser-Ethylenglykol-Gemisches abgetrennt. Nach dem Abkühlen wird mit Sodalösung gewaschen und über Natriumsulfat getrocknet. Man fügt 3 g Soda hinzu und destilliert an einer 30-cm Füllkörperkolonne. Man erhält 103 g Produkt, das zu 26% aus Isomer 1 und zu 43% aus Isomer 2 besteht.
Geruch: wie unter a) angegeben, jedoch weniger Impact und Charakter
c) Reaktionsparameter: Schleppmittel: Cyclohexan; Menge p-Toluolsulfonsäure = 1 g
Molverhältnis 2-Methyl-4-(2.6.6-trimethyl-1-cyclohexen-1-yl)-2-butenal:Ethylenglykol = 1:3
103g (0,5 Mol) 2-Methyl-4-(2.6.6-trimethyl-1-cyclohexen-1-yl)-2-butenal, 93g (1,5 Mol) Ethylenglykol, 1 g p-Toluolsulfonsäure werden mit 200g Cyclohexan 14 Stunden am Wasserabscheider erhitzt. Nach dem Abkühlen wird mit Sodalösung gewaschen und über Natriumsulfat getrocknet. Man fügt 2,6 g Soda hinzu und destilliert an einer 20-cm Füllkörperkolonne. Es werden 90 g Produkt gewonnen, das zu 43% aus Isomer 1 und zu 22% aus Isomer 2 besteht.
d) Reaktionsparameter: Schleppmittel: Toluol; Menge p-Toluolsulfonsäure = 1 g
Molverhältnis 2-Methyl-4-(2.6.6-trimethyl-1-cyclohexen-1-yl)-2-butenal:Ethylenglykol = 1:1,3
103 g (0,5 Mol) 2-Methyl-4-(2.6.6-trimethyl-1-cyclohexen-1-yl)-2-butenal, 40,3 g (0,65 Mol) Ethylenglykol und 1 g p-Toluolsulfonsäure werden mit 200 g Toluol 2,5 Stunden am Wasserabscheider erhitzt. Hierbei werden 25 g eines Wasser-Ethylenglykol-Gemisches abgetrennt. Nach dem Abkühlen wird mit Sodalösung gewaschen, über Natriumsulfat getrocknet und das Toluol am Rotationsverdampfer abdestilliert. Anschließend destilliert man am Dünnschichtverdampfer (Manteltemperatur = 197°C, Druck = 1,6 mbar) bei einer Kopftemperatur von 125-140°C 98,5 g Rohprodukt ab, in dem 58,5% Isomer 1 und 7,8% Isomer 2 enthalten sind. Zum Rohdestillat fügt man 3 g Soda hinzu und destilliert an einer 20-cm Füllkörperkolonne. Bei 121-125°C/1,5-1,9 mbar resultieren 65 g Produkt, das zu 72% aus Isomer 1 und zu 8,4% aus Isomer 2 besteht, und das weitere Doppelbindungsisomere enthält.
e) Umisomerisierung
13 g eines Isomerengemisches, in dem 26% Isomer 1 und 52% I-somer 2 enthalten sind, werden mit 0,1 g p-Toluolsulfonsäure in 20 g Toluol 15 Stunden unter Rückfluss erhitzt. Nach dem Abkühlen wird mit Sodalösung gewaschen und über Natriumsulfat getrocknet. Mittels Kugelrohrdestillation werden 9,5 g Isomerengemisch erhalten, das zu 70% aus Isomer 1 und zu 7,5% aus Isomer 2 besteht.

### Beispiel 2

### Herstellung eines Isomerengemisches mit 2-[1-Methyl-3-(2,6,6-trimethyl-1-cyclohexen-1-yl)-allyl]- 1,3-Dioxan als Hauptkomponente

103g (0,5 Mol) 2-Methyl-4-(2.6.6-trimethyl-1-cyclohexen-1-yl)-2-butenal, 38 g (0,5 Mol) 1,3-Propandiol und 1 g p-Toluolsulfonsäure werden mit 200 g Toluol 2,5 Stunden am Wasserabscheider erhitzt. Nach dem Abkühlen wird mit Sodalösung gewaschen und über Natriumsulfat getrocknet. Man fügt 3 g Soda hinzu und destilliert an einer 30-cm Füllkörperkolonne. Bei 122-126°C/1,6-0,82 mbar resultieren 81 g Produkt, das zu 66% aus 2-[1-Methyl-3-(2,6,6-trimethyl-1-cydohexen-1-yl)-allyl]- 1,3-Dioxan besteht und daneben weitere Doppelbindungsisomere enthält.

Geruch: Pflaume, Feige, Iris, Liebstock, Tee, würzig

Die Strukturbestimmung erfolgte mittels NMR-Spektroskopie

### 2-[1-Methyl-3-(2,6,6-trimethyl-1-cydohexen-1-yl)-altyl]- 1,3-Dioxan

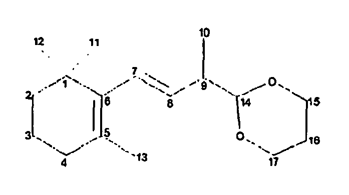

| δ(ppm) | | | J (Hz) | |
|---|---|---|---|---|
| 0,97 | s | 3H | | CH3 (11 oder 12) |
| 0,98 | s | 3H | | CH3 (11 oder 12) |
| 1,07 | d | 3H | 6,9 | CH3 (10) |
| 1,31 | d br | 1H | 13,3 | CH (16) |
| 1,43 | m | 2H | | CH2 (2) |
| 1,58 | m | 2H | | CH2 (3) |
| 1,67 | q | 3H | 0,9 | CH3 (13) |
| 1,95 | t | 2H | 6,3 | CH2 (4) |
| 2,06 | ttd | 1H | 5,1/ 12,4/ 13,3 | CH (16) |
| 2,41 | dqd | 1H | 4,9/ 6,9/ 8,0 | CH (9) |
| 3,74 | dt | 2H | 2,6/ 12,1 | CH2 (15,17) |
| 4,11 | m | 2H | | CH2 (15,17) |
| 4,36 | d | 1H | 4,9 | CH (14) |
| 5,36 | dd | 1H | 8,0/ 16,0 | CH (8) |
| 5,87 | d br | 1H | 16,0 | CH (7) |

Lösungsmittel: CDCl₃
interner Standard: Tetramethylsilan (TMS)
Frequenz: 400 MHz

### Beispiel 3:

Erfindungsgemäße Riechstoffkomposition im Vergleich mit einer Basiskomposition:
3.1 Basiskomposition:

| | |
|---|---|
| | Gew.-Teile |
| Aldehyd C 7 50% in PEA | 1 |
| Aldehyd C 8 | 0,7 |
| Aldehyd C 9 | 1,2 |
| Aldehyd C 10 | 1,5 |
| Alkohol C 6 Kosher | 2 |
| Cire D'Abeille Abs. | 2 |
| Citral FF | 8 |
| Citronellal Supra | 0,9 |
| Citronellol Laevo | 135 |
| Citronellylacetat Extra | 5 |
| Citronellylformiat | 2 |
| Citronenoel Terpene Wonf | 2 |
| Dipropylenglykol | 518,1 |
| Estragol | 7 |
| Ethylheptylat | 1 |
| Ethylrizinoleat | 15 |
| Eugenol | 2 |
| Famesol 100% | 3 |
| Geranitril | 20 |
| Geraniumoel Bourbon | 10 |
| Geranylformiat Supra | 1 |
| Linalool | 5 |
| Myristinsäure | 10 |
| Nonylacetat | 2 |
| Octylacetat-1 | 2 |
| Phenylacetaldehyd 50% DPG 2% DPG | 5 |
| Phenylethylacetat | 15 |
| Phenylethylformiat | 4 |
| Propylad | 10 |
| Rosenoxid L | 0,3 |
| Terpinenol-4 nat | 0,3 |
| Tetrahydrogeraniol | 8 |

3.2 Die Zugabe von 200 g Produkt aus Beispiel 1 zu der Basiskomposition gemäß 3.1 bewirkt einen frischen, natürlichen und angenehmen Blüteneffekt. Die fruchtigen Elemente wirken abrundend und feminin, so dass ein sanfter Akkord resultiert.

## Patentansprüche

1. Acetal der Formel I 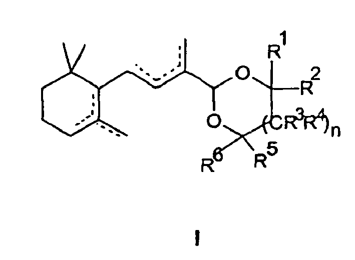 wobei
die Reste R¹ bis R⁶ unabhängig voneinander jeweils Wasserstoff, Methyl, Ethyl, n-Propyl, iso-Propyl, n-Butyl, iso-Butyl, sec-Butyl oder tert.-Butyl
n = 0 oder 1 ist und
am Ort einer gestrichelt dargestellten Linie zwischen zwei C-Atomen eine Einfachbindung oder Doppelbindung vorliegt,
wobei eine gegebenenfalls vorhandene Doppelbindung in der den aliphatischen Ring mit der Acetal-Gruppe verbindenden Kette E- oder Z-konfiguriert ist,
mit der Maßgabe, dass
- die Zahl der Doppelbindungen, an denen ein C-Atom des aliphatischen Rings beteiligt ist, 0 oder 1 ist
- die Zahl der Doppelbindungen, an denen kein C-Atom des aliphatischen Rings beteiligt ist, 0 oder 1 ist.

2. Acetal nach Anspruch 1, wobei
die Reste R¹ bis R⁶ unabhängig voneinander jeweils Wasserstoff oder Methyl sind.

3. Acetal nach Anspruch 1 oder 2, wobei das Acetal der nachfolgenden Formel IA oder IB entspricht: 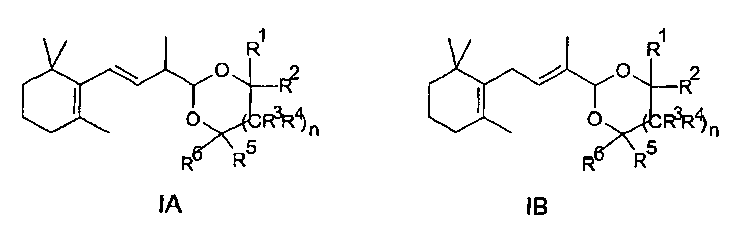 wobei die jeweilige Doppelbindung in der den aliphatischen Ring mit der Acetal-Gruppe verbindenden Kette E- oder Z-konfiguriert ist.

4. Acetal nach Anspruch 3, wobei das Acetal
2-[1-Methyl-3-(2,6,6-trimethyl-1-cydohexen-1-yl)-allyl]-1,3-Dioxolan
oder
2-[1-Methyl-3-(2,6,6-trimethyl-1-cyclohexen-1-yl)-propenyl]-1,3-Dioxolan
ist.

5. Acetal nach einem der Ansprüche 1-4, herstellbar durch Acetalisierung von 2-Methyl-4-(2,6,6-trimethyl-1-cyclohexen-1-yl)-2-butenal.

6. Mischung von zwei oder mehr Acetalen nach einem der Ansprüche 1-4.

7. Verwendung eines Acetals nach einem der Ansprüche 1-5 oder einer Mischung von zwei oder mehr Acetalen nach Anspruch 6 (a) als Riechstoff oder (b) zur Herstellung einer Riechstoffmischung oder eines Parfüms.

8. Produkt, umfassend
- einen Träger oder ein Substrat sowie
- eine damit in direktem Kontakt stehende sensorisch wirksame Menge eines Acetals nach einem der Ansprüche 1-5 oder einer Mischung von zwei oder mehr Acetalen nach Anspruch 6.

9. Produkt nach Anspruch 8, ausgewählt aus der Gruppe bestehend aus: alkoholischen Parfüms, Körperpflegeprodukten und im Haushalt zu verwendenden Reinigungs- oder Pflegeprodukten.

10. Produkt nach Anspruch 9, **dadurch gekennzeichnet, dass** die Körperpflegeprodukte ausgewählt sind aus der Gruppe bestehend aus Seifen, Duschgelen, Shampoos, Badezusätzen, Hautcremes, Körperlotionen und Deodorantien, und die Reinigungsmittel ausgewählt sind aus der Gruppe bestehend aus Waschmitteln, Wäscheweichspülern, Raumluftverbesserem und Reinigern.

11. Verfahren zur Herstellung eines Acetals nach einem der Ansprüche 1-5 oder einer Mischung nach Anspruch 6, **dadurch gekennzeichnet, dass** 2-Methyl-4-(2,6,6-trimethyl-1-cyGohexen-1-yl)-2-butenal mit einem aliphatischen 1,2- oder 1,3-Diol unter Säurekatalyse und Wasserabscheidung umgesetzt wird.

12. Verfahren nach Anspruch 9, wobei das bei der Reaktion gebildete Wasser entfernt wird durch (a) Destillation, (b) azeotrope Destillation oder (c) azeotrope Destillation gemeinsam mit einem Schleppmittel.

## Claims

1. Acetal of formula I: 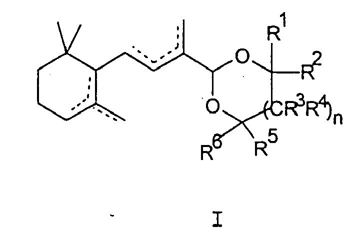 in which
the radicals R¹ to R⁶ independently of one another are each hydrogen, methyl, ethyl, n-propyl, isopropyl, n-butyl, isobutyl, sec-butyl or tert-butyl,
n = 0 or 1, and
there is a single bond or double bond at the location of a broken line between two C atoms,
any double bond present in the chain linking the aliphatic ring to the acetal group having the E or Z configuration,
with the proviso that
- the number of double bonds incorporating a C atom of the aliphatic ring is 0 or 1, and
- the number of double bonds not incorporating a C atom of the aliphatic ring is 0 or 1.

2. Acetal according to Claim 1 in which the radicals R¹ to R⁶ independently of one another are each hydrogen or methyl.

3. Acetal according to Claim 1 or 2 which has formula IA or IB below: 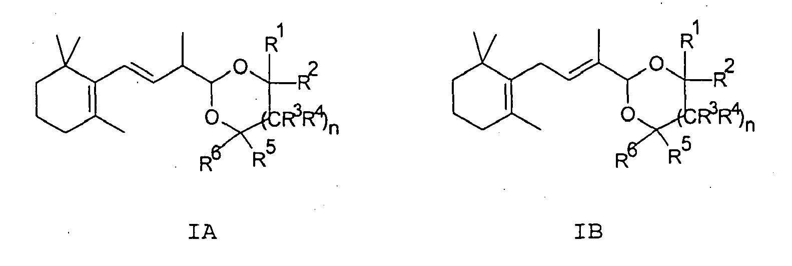 the double bond in the chain linking the aliphatic ring to the acetal group having the E or Z configuration in each case.

4. Acetal according to Claim 3 which is 2-[1-methyl-3-(2,6,6-trimethyl-1-cyclohexen-1-yl)allyl]-1,3-dioxolane
or
2-[1-methyl-3-(2,6,6-trimethyl-1-cyclohexen-1-yl)propenyl]-1,3-dioxolane.

5. Acetal according to one of Claims 1-4 which can be prepared by the acetalization of 2-methyl-4-(2,6,6-trimethyl-1-cyclohexen-1-yl)-2-butenal.

6. Mixture of two or more acetals according to one of Claims 1-4.

7. Use of an acetal according to one of Claims 1-5 or a mixture of two or more acetals according to Claim 6 (a) as a fragrance, or (b) for the preparation of a mixture of fragrances or a perfume.

8. Product comprising
- a carrier or a substrate, and
- a sensorially effective amount, in direct contact therewith, of an acetal according to one of Claims 1-5 or a mixture of two or more acetals according to Claim 6.

9. Product according to Claim 8 selected from the group comprising alcoholic perfumes, body care products and household cleaning or care products.

10. Product according to Claim 9, **characterized in that** the body care products are selected from the group comprising soaps, shower gels, shampoos, bath additives, skin creams, body lotions and deodorants, and the cleaning products are selected from the group comprising detergents, fabric softeners, air purifiers and cleaners.

11. Process for the preparation of an acetal according to one of Claims 1-5 or a mixture according to Claim 6, **characterized in that** 2-methyl-4-(2,6,6-trimethyl-1-cyclohexen-1-yl)-2-butenal is reacted with an aliphatic 1,2- or 1,3-diol under acid catalysis and water separation.

12. Process according to Claim 9 wherein the water formed in the reaction is removed by (a) distillation, (b) azeotropic distillation, or (c) azeotropic distillation together with an entraining agent.

## Revendications

1. Acétals de formule I 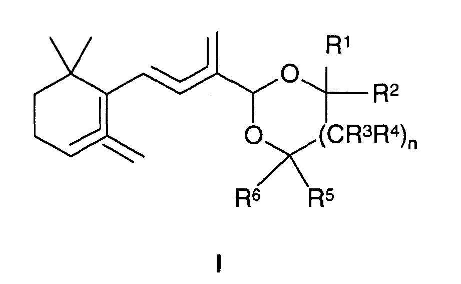 dans laquelle
R¹ à R⁶ représentent chacun, indépendamment les uns des autres, l'hydrogène, un groupe méthyle, éthyle, n-propyle, isopropyle, n-butyle, isobutyle, sec-butyle ou tert-butyle,
n est égal à 0 ou 1 et
un trait en pointillés reliant deux atomes de carbone indique une liaison simple ou une double liaison,
une double liaison éventuellement présente dans la chaîne reliant le cycle aliphatique au groupe acétal est en configuration E ou Z,
sous réserve que
- le nombre des doubles liaisons auxquelles un atome de carbone du cycle aliphatique participe est égal à 0 ou 1,
- le nombre des doubles liaisons auxquelles aucun atome de carbone du cycle aliphatique ne participe est égal à 0 ou 1.

2. Acétal selon la revendication 1, pour lequel
R¹ à R⁶ représentent chacun, indépendamment les uns des autres, l'hydrogène ou un groupe méthyle.

3. Acétal selon la revendication 1 ou 2, répondant à l'une des formules IA ou IB ci-après : 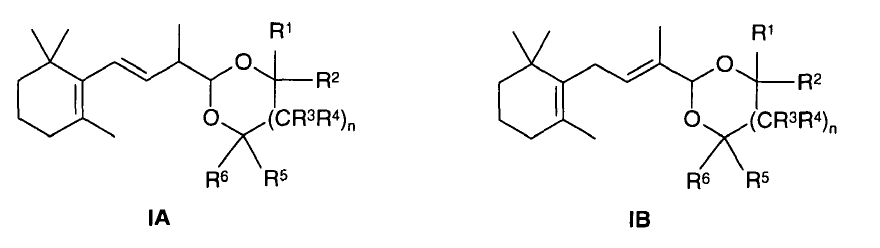 chacune des doubles liaisons présentes dans la chaîne reliant le cycle aliphatique au groupe acétal étant en configuration E ou Z.

4. Acétal selon la revendication 3, consistant en
le 2-[1-méthyl-3-(2,6,6-triméthyl-1-cyclohexén-1-yl)-allyl]-1,3-dioxolanne
ou en
le 2-[1-méthyl-3-(2,6,6-triméthyl-1-cyclohexén-1-yl)-propényl]-1,3-dioxolanne.

5. Acétal selon l'une des revendications 1 à 4, préparé par acétalisation du 2-méthyl-4-(2,6,6-triméthyl-1-cyclohexén-1-yl)-2-buténal.

6. Mélange de deux acétals ou plus selon l'une des revendications 1 à 4.

7. Utilisation d'un acétal selon l'une des revendications 1 à 5 ou d'un mélange de deux acétals ou plus selon la revendication 6, (a) en tant que matière odorante ou (b) pour la préparation d'un mélange de matières odorantes ou d'un parfum.

8. Produit, comprenant
- un support ou un substrat et
- en contact direct avec celui-ci, un acétal selon l'une des revendications 1 à 5 ou un mélange de deux acétals ou plus selon la revendication 6, en quantité sensorielle efficace.

9. Produit selon la revendication 8 choisi dans le groupe consistant en : les parfums alcooliques, les produits pour soins corporels et les produits de nettoyage ou de soins à usage ménager.

10. Produit selon la revendication 9, **caractérisé en ce que** les produits pour soins corporels sont choisis dans le groupe consistant en les savons, les gels pour douche, les shampooings, les additifs pour bains, les crèmes pour la peau, les lotions corporelles et les désodorisants, et les produits de nettoyage sont choisis dans le groupe consistant en les produits de lavage, les produits de rinçage adoucissants du linge, les assainisseurs d'air et les détergents.

11. Procédé pour la préparation d'un acétal selon l'une des revendications 1 à 5 ou d'un mélange selon la revendication 6, **caractérisé en ce que** l'on fait réagir le 2-méthyl-4-(2,6,6-triméthyl-1-cyclohexén-1-yl)-2-buténal avec un 1,2- ou 1,3-diol aliphatique avec catalyse acide et séparation d'eau.

12. Procédé selon revendication 9 dans lequel l'eau formée dans la réaction est éliminée (a) par distillation, (b) par distillation azéotropique ou (c) par distillation azéotropique avec un agent d'entraînement.
